(19) Europäisches Patentamt
European Patent Office
Office européen des brevets

(11) **EP 4 268 778 A1**

(12) **EUROPEAN PATENT APPLICATION**

(43) Date of publication:
**01.11.2023 Bulletin 2023/44**

(21) Application number: **22173885.9**

(22) Date of filing: **17.05.2022**

(51) International Patent Classification (IPC):
*A61F 13/15* (2006.01)   *A61F 13/49* (2006.01)
*A61F 13/505* (2006.01)   *A61F 13/58* (2006.01)
*A61F 13/62* (2006.01)   *A61F 13/74* (2006.01)

(52) Cooperative Patent Classification (CPC):
**A61F 13/15268; A61F 13/49003; A61F 13/505;**
**A61F 13/58; A61F 13/62; A61F 13/74**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **29.04.2022 EP 22170752**

(71) Applicants:
• **Ontex BV**
**9255 Buggenhout (BE)**

• **Ontex Group NV**
**9320 Erembodegem (BE)**

(72) Inventors:
• **IDELSON, Alissa**
**53359 Rheinbach (DE)**
• **WEBER, Ainas**
**53474 Bad Neuenahr-Ahrweiler (DE)**
• **JANSEN, Bart**
**2640 Mortsel (BE)**

(74) Representative: **Macchetta, Andrea**
**Ontex BV**
**Korte Keppestraat 21**
**9320 Erembodegem-Aalst (BE)**

(54) **RE-USABLE SHELL AND DISPOSABLE INSERT WITH IMPROVED FASTENING MEANS**

(57)     A patch comprising: a base layer including a body-facing surface and a garment-facing surface; the base layer comprising a perimeter formed by a first and second transverse edge and first and second longitudinal edges connecting the first and second transverse edges; the garment-facing surface comprising a fastening means for releasable attachment to a re-usable outer shell, the body-facing surface comprising a fastening means for releasable attachment to a disposable insert.

**FIG. 4**

## Description

### TECHNICAL FIELD

The disclosure relates to absorbent articles such as disposable absorbent articles, preferably selected from the group consisting of diapers (whether for baby or adults), pants (whether for baby or adults), briefs, and combinations thereof generally of the hybrid type i.e. a re-usable and/or washable outer shell and a disposable (generally single-use) absorbent insert cooperable with said outer shell.

### BACKGROUND

Hybrid-type articles, comprising a re-usable outer shell and disposable absorbent insert, are becoming more and more sought after in the market in view of their sustainable benefits and reduced waste production.
Different designs are available for such products such as described in EP 3 842 017 A1 wherein the outer shell is generally a pant-type component that may comprise elastic components therein and the disposable absorbent insert comprises liquid permeable topsheet, liquid impermeable backsheet and absorbent core sandwiched therebetween and is generally re-fastenably joinable to a crotch region of the outer shell.
Other examples are described in EP 3 659 563 A1 where the disposable insert is re-fastenably joined to the outer shell and the outer shell is in the form of a re-fastenable diaper pant; and EP 3 895 673 A1 wherein the disposable inserts are biodegradable and/or compostable.
Disadvantages of such devices include risk of detachment upon movement of the wearer, and risk of soiling clothes and other surfaces during the disposal thereof. Another disadvantage of current hybrid concepts is the use of a liquid impervious pouch. These 3-in-1 concepts include an extra component having a negative environmental impact. A further disadvantage exists when the disposable insert is applied onto the re-usable outer shell through the use of adhesive. Such hybrid concept typically uses a release tape which needs to be removed before applying the insert on the shell. This release tape adds to the negative environmental impact because it is typically made of plastic components.
Thus, although there have already been significant advancements in this field, there still remains a need to further improve hybrid-type articles so as to provide improved attachment of the disposable insert onto the outer shell to keep the insert in place upon movement of the wearer. A further need exists to allow for the wearer or caregiver to more easily or more efficiently wash and store the re-usable outer shell and to further improve the sustainable aspects of such hybrid-type articles.

## SUMMARY

In a first aspect, the disclosure relates to a patch comprising: a base layer including a body-facing surface and a garment-facing surface; the base layer comprising a perimeter formed by a first and second transverse edge and first and second longitudinal edges connecting the first and second transverse edges; the garment-facing surface comprising a fastening means for releasable attachment to a re-usable outer shell, the body-facing surface comprising a fastening means for releasable attachment to a disposable insert.
In a second aspect, the disclosure relates to a re-usable outer shell comprising: a front and back waist region having uppermost and lowermost edges; and a crotch region interposed between the front and back waist region, wherein the uppermost edges of the waist region form a waist opening and the lowermost edges of the waist region together with lateral extremities of the crotch region form two oppositely disposed leg openings; and wherein at least the body-facing surface of the front (F) and/or back (B) waist region(s) is adapted for receiving one or more discrete patched; and wherein the re-usable outer shell comprises fastening means on the body-facing surface including one or more snap fasteners selected from male snap fasteners and/or female snap fasteners, for releasably engaging female and/or male snap fastener(s) of the discrete patches.
In a further aspect, the disclosure relates to an absorbent assembly comprising one or more patches, a re-usable outer shell and a disposable insert.
In a further aspect, the disclosure relates to a kit of parts comprising a plurality of patches and at least one of:
(i) one or more outer shells, forming a plurality of absorbent assemblies; preferably wherein the plurality of inserts differ in at least one of: size, absorbency, biodegradable and/or compostable content, shape, and number of components contained therein; and (ii) one or more inserts forming a part of a plurality of absorbent assemblies; one or more outer shells; preferably wherein the plurality of outer shells differ in at least one of: size, absorbency, biodegradable and/or compostable content, shape, and number of components contained therein.

### BRIEF DESCRIPTION OF THE FIGURES

Fig. 1 Is an illustration, cross-section view and bottom planar view, of a patch according to an embodiment herein.

Fig. 2 Is an illustration, top planar view, of a patch according to an embodiment herein, showing an exemplary alternating arrangement of fastening means and loop components.

Fig. 3 and 4 Are illustrations, garment-facing side, of fastening means according to an embodiment herein.

Fig. 5 and 6 Are illustrations of a fiber fastener according to an embodiment of the disclosure. With Fig. 5 showing a plurality of fiber fasteners on a patch or tape and Fig. 8 an exemplary individual fiber fastener.

Fig. 7 Is an illustration, top-planar view (body-facing surface), of a re-usable outer shell according to an embodiment herein.

Fig. 8 Is an illustration, cross-section view, of an insert according to an embodiment herein.

Fig. 9 Is an illustration, bottom planar view (garment-facing side), of an insert according to an embodiment herein.

Fig. 10 and 11 Are illustrations, cross-section view, of a Z-folded fastening means according to an embodiment herein.

Fig. 12 Is a simplified illustration, cross-section view, of an insert according to an embodiment herein.

Fig. 13 Illustrates an exemplary process for the production of high-AUL Bio-SAP.

**Fig. 14** Is an illustration, top planar view (body-facing side), of an insert with channel(s) according to an embodiment herein.

**Fig. 15** Is an illustration, top planar view (body-facing side), of a re-usable outer shell with one or more patches according to an embodiment herein.

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of' as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", "nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within $\pm45°$ of the lateral direction.

The term "longitudinal" refers to a direction running parallel to the maximum linear dimension of the article and when combined with the term "substantially" includes directions within $\pm45°$ of the longitudinal direction.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

THE PATCH

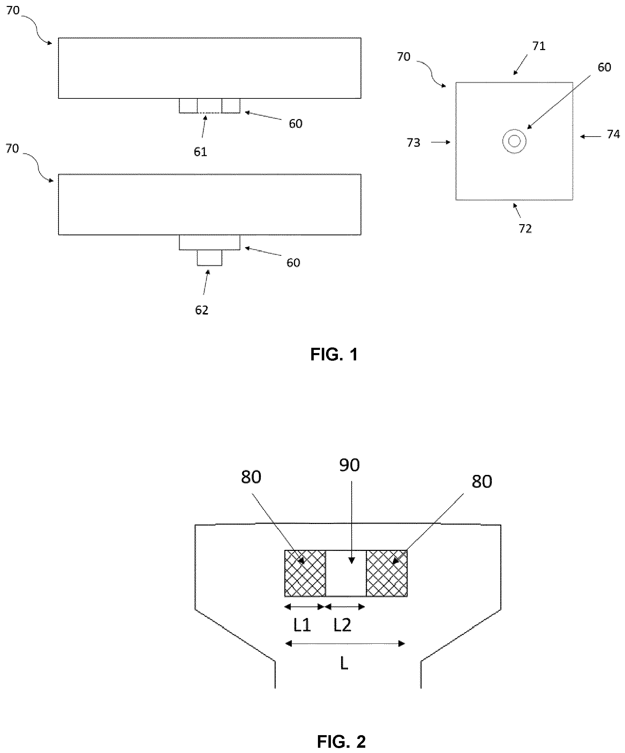

As shown in Fig.1, patches (70) herein typically comprise a base layer including a body-facing surface and a garment-facing surface. Generally, the base layer comprises a perimeter formed by a first and second transverse edge (71 and 72) and first and second longitudinal edges (73 and 74) connecting the first and second transverse edges. The garment-facing surface, of the patches herein, comprises a fastening means (60) for releasable attachment to a re-usable outer shell (20), the body-facing surface comprises a fastening means (80) for releasable attachment to a disposable insert (1). Advantageously, the use of such patches (70) avoids the necessity of a fluid impervious pouch which has a negative impact on the sustainable nature of the entire product concept. It would lead to the use of less plastic by replacing the fluid impervious pouch by one or more patches described herein which allow for a refastenable connection between disposable insert and re-usable outer shell in an intuitive and efficient manner.

In a preferred embodiment, as shown in Fig.1, the fastening means (60), positioned on the garment-facing surface of the one or more patches (70), includes one or more snap fasteners, selected from male snap fasteners (62) and/or female snap fasteners (61). Advantageously, the use of snap fasteners allows for an efficient refastenable connection with a re-usable outer shell (20) through a 'lock-and-key' principle. The connection is made by the complementarity between male snap fasteners (62) and female snap fasteners (61). The lock-and-key principle ensures correct positioning of the one or more patches (70) with very high reproducability.

In an embodiment, as shown in Fig. 2, the one or more patches (70) comprise alternating zones of fastening means (80) and loop components (90), wherein the length ratio (L1/L) of the fastening means (80) is more than about 0,1 and wherein the length ratio (L2/L) of the loop components (90) is more than about 0,1. The length ratio (L1/L) of the fastening means (80) preferably is from about 0,1 to about 0,9; even more preferably from about 0,2 to about 0,8; still more preferably from about 0,25 to about 0,8; particularly preferably from about 0,3 to about 0,75; particularly more preferably from about 0,35 to about 0,7; most preferably from about 0,4 to about 0,7. The length ratio (L1/L) of the loop components (90) preferably is from about 0,1 to about 0,9; even more preferably from about 0,2 to about 0,8; still more preferably from about 0,2 to about 0,75; particularly preferably from about 0,25 to about 0,7; particularly more preferably from about 0,3 to about 0,65; most preferably from about 0,3 to about 0,6.

As used herein, the term "loop" or "loop component" or "loop-type" refers to a hook-and-loop fastener female member including an engaging layer engageable with a male member (sometimes referred to as "mechanical hook member"). It is intended to mean any fibrous material which can mechanically engage with the hook-type material. The hook-and-loop fastener female member may comprise a physical property layer (layer to be arranged mainly in order to support or reinforce the engaging layer, carry out fixing or adhesion to another article, or form a printed layer on the female member).

The thickness of the hook-and-loop fastener female member may be set to any appropriate thickness depending on the purpose. The number of layers of the engaging layer may be only one, or may be two or more.

Suitable loop-type materials are formed by nonwoven materials. The nonwoven materials can be extensible, elastic, or nonelastic. Suitable nonwoven materials include spunbond nonwovens, fluffy non-woven fabrics (e.g., a nonwoven fabric obtained by a thermal bonding method, a spunlace method, or an air-through bonding method), meltblown nonwovens, carded nonwovens, thermobonded nonwovens, air-through bonded nonwovens, flashspun nonwoven webs, electrospun nonwoven webs and hydroentangled nonwovens, and combinations thereof. The nonwoven materials can comprise an embossing pattern. Other suitable loop-type materials may comprise woven or knitted materials. The loop-type materials are formed of conventional fibers including nylon, polyester, polypropylene, polyethylene or other like synthetic or natural fibers. The fibers may be homogeneous structural bodies or may contain bicomponent structural bodies. The fibers forming the non-woven fabric included in the engaging layer may contain any appropriate other component as long as the functionality of the engaging layer is not impaired. Examples of such components are other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. They may be used alone or in combination. The basis weight of the non-woven fabric in the engaging layer is preferably from 20 gsm to 60 gsm, more preferably from 20 gsm to 50 gsm, still more preferably from 20 gsm to 40 gsm, particularly preferably from 25 gsm to 40 gsm, most preferably from 30 gsm to 40 gsm.

In a preferred embodiment, the fastening means (80), positioned on the body-facing surface of the one or more patches (70), comprises one or more mechanical areas (A2) and/or one or more adhesive areas (A1). Having such exposed adhesive area has a positive impact on peel strength and dynamic shear stress. Exposed adhesive area significantly increases the initial dynamic shear, but is not further increasing by maximizing the exposed adhesive area, in which the mode of failure 'delamination of the nonwoven from the PE film' will be a significant contributing factor. Preferably, the total area ratio ($\Sigma$A1 / $\Sigma$A2) of the fastening means is from about 0,4 to about 1, more preferably from about 0,45 to about 0,8 and even more preferably about 0,5.

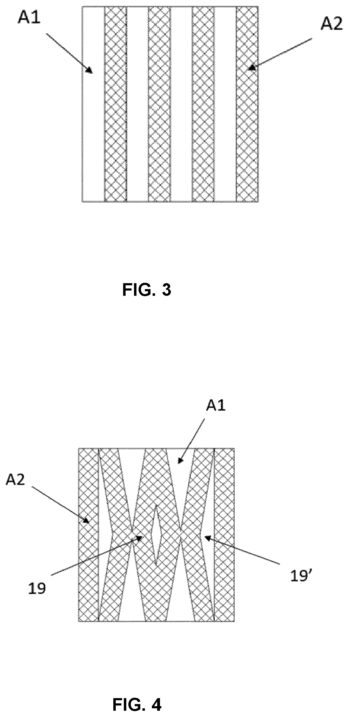

In an embodiment, as shown in Fig.3 and Fig.4, the one or more mechanical areas (A2) can be alternatingly arranged with the one or more adhesive areas (A1). The mechanical areas (A2) can take the form of lines being straight, curved or sinusoidally shaped. The mechanical areas (A2) alternatively can form a mesh structure (19) in which discrete adhesive patches (19') are positioned in the intermediate spaces.

In an embodiment the one or more mechanical areas comprise hooks. In an alternative embodiment the one or more mechanical areas comprise fiber fasteners.

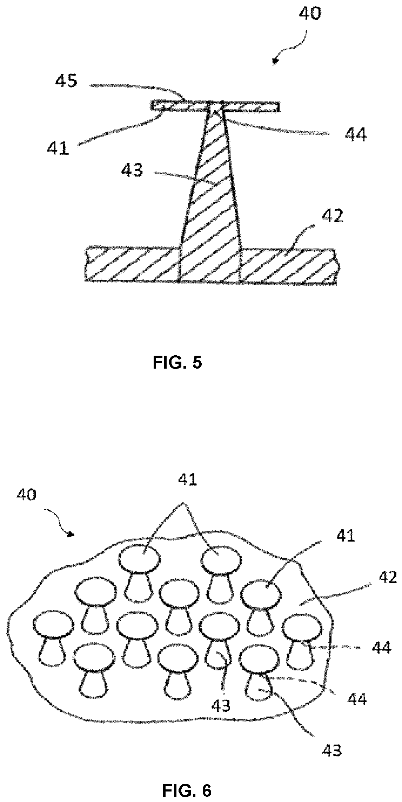

Preferably, as shown in Figs. 5 and 6, the fiber fastener (40), when used, comprises a plurality of bonding elements spaced apart from one another, said bonding elements each comprising a head part (41), a backing part (42) and a stem part (43) connecting the head part (41) and the backing part (42), preferably integrally connected together, and extending therebetween, and wherein the head part (41) consists of a head plate, the diameter of which may be greater than the diameter on every point of the stem part (43) that is conical in form, wherein the stem part (43) is connected flexibly to the head part by means of an articulation part (44), and that a contact surface (45) of the head part (41) enables detachable adhesion between the disposable insert (1) and the re-usable outer shell (20). Advantageously, this arrangement allows for better joining of the substrates compared to known hook and loop (or Velcro®) type fasteners.

"Fiber fasteners" as used herein generally means fasteners wherein the maximum diameter of the stem part is less than 50 $\mu$m, preferably from 10 $\mu$m to 40 $\mu$m, more preferably from 15$\mu$m to 30 $\mu$m, and typically wherein the minimum diameter of the stem part (53) is less than 30 $\mu$m, preferably from 1 $\mu$m to 15 $\mu$m, more preferably from 5 $\mu$m to 9 $\mu$m, and wherein the ratio of the perimeter of the contact surface (55) to the maximum diameter of the stem may be from 0.5 to 2, preferably from 0.8 to 1.5, more preferably from 0.9 to 1.4. Commercially available fiber fasteners suitable for use herein comprise the micro-structured silicone fastener film manufactured and supplied by Gottlieb Binder GmbH u. Co KG, for example under the Gecko® brand such as Gecko® Nanoplast® tape.

THE OUTER SHELL

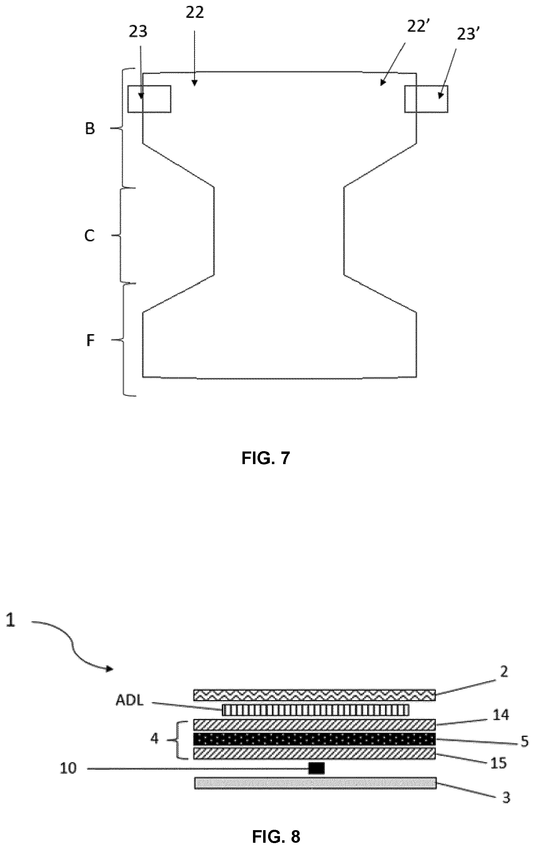

As exemplified in Fig. 7, re-usable outer shells (20) herein preferably comprise: a front (F) and back (B) waist region having uppermost and lowermost edges; and a crotch region (C) interposed between the front and back waist region (F, B), wherein the uppermost edges of the waist region form a waist opening and the lowermost edges of the waist region together with lateral extremities of the crotch region form two oppositely disposed leg openings; and wherein at least the body-facing surface of the crotch region (C) is adapted for receiving an absorbent insert (1) as described herein; and wherein at least the body-facing surface of the front (F) and/or back (B) waist region(s) is adapted for receiving one or more discrete patches (70) as described herein.

In a preferred embodiment, the re-usable outer shell (20) comprises fastening means (30) on the body-facing surface including one or more snap fasteners (60), selected from male snap fasteners (62) and/or female snap fasteners (61), for releasably engaging female and/or male snap fastener(s) of the discrete patches (70). Advantageously, the use of snap fasteners allows for an efficient refastenable connection with a re-usable outer shell (20) through a 'lock-and-key' principle. The connection is made by the complementarity between male snap fasteners (62) and female snap fasteners (61). The lock-and-key principle ensures correct positioning of the one or more patches (70) with very high reproducibility and thus acts as a positioning indicator.

The re-usable outer shell (20) may comprise re-fastenable or permanent side seams joining the front and back waist regions, preferably in the form of a pant; or the back waist region (B), as shown in Fig.5 comprises transversely extending side panels (22, 22') each comprising a fastener (23, 23') for coupling to a landing zone positioned at a garment facing side of the front waist region (F), preferably in the form of a diaper.

In an embodiment, the re-usable outer shell (20) comprises one or more male snap fasteners (62) and the back waist region (B) comprises one or more female snap fasteners (61). In an alternative embodiment the front waist region (F) comprises one or more female snap fasteners (61) and the back waist region comprises one or more male snap fasteners (62). Advantageously, this allows for the user to connect the front waist region (F) and the back waist region (B) to each other upon washing of the re-usable outer shell (20) or when the re-usable outer shell (20) is not being used. With this arrangement, the re-usable outer shell (20) can be stored in a compact manner.

THE ABSORBENT ASSEMBLY

Absorbent assemblies herein may comprise one or more patches (70) described herein coupled to an outer shell (20) as described herein and coupled to an insert (1) as described below.

As exemplified in Fig. 8, inserts (1) herein typically comprise a liquid permeable topsheet (2), a backsheet (3); and an absorbent core (4) comprising absorbent material (5) therein and being sandwiched between said topsheet (2) and backsheet (3).

Inserts herein may further comprise an acquisition distribution layer (ADL) positioned between the topsheet and the absorbent core, preferably between the topsheet and the top core wrap layer. The acquisition distribution layer may have a basis weight of from 15 gsm to 55 gsm, preferably from 18 gsm to 50 gsm, even more preferably from 19 gsm to 45 gsm. Preferably the acquisition distribution layer is selected from a carded air-through bonded nonwoven, a carded thermobonded calendered nonwoven, a spunbond nonwoven, and combinations thereof.

Generally, the insert (1) comprises a perimeter formed by first and second transverse edges (6, 7) and first and second longitudinal edges (8, 9) connecting the first and second transverse edges (6, 7); a longitudinal centerline extending (y) substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7).

In an embodiment, the insert comprises a fastening means (10), as shown in Fig.6, disposed on the garment-facing side of the backsheet (3) being releasably attachable to a re-usable outer shell (20). The fastening means (10) preferably comprise one or more adhesive areas (A1') and one or more mechanical areas (A2'). Having such exposed adhesive area has a positive impact on peel strength and dynamic shear stress. Exposed adhesive area significantly increases the initial dynamic shear, but is not further increasing by maximizing the exposed adhesive area, in which the mode of failure 'delamination of the NW from the PE film' will be a significant contributing factor. Preferably, the total area ratio ($\Sigma A1'$ / $\Sigma A2'$) of the fastening means is from about 0,4 to about 1, more preferably from about 0,45 to about 0,8 and even more preferably about 0,5.

Inserts (1) herein are releasably attachable to the one or more patches (70) as described herein and/or are releasably attachable to the re-usable outer shell (20).

In an embodiment, the one or more mechanical areas (A2') are alternatingly arranged with the one or more adhesive areas (A1'). The mechanical areas (A2') can take the form of lines being straight, curved or sinusoidally shaped. In an alternative embodiment, the mechanical areas (A2') form a mesh structure (19) in which discrete adhesive patches (19') are positioned in the intermediate spaces.

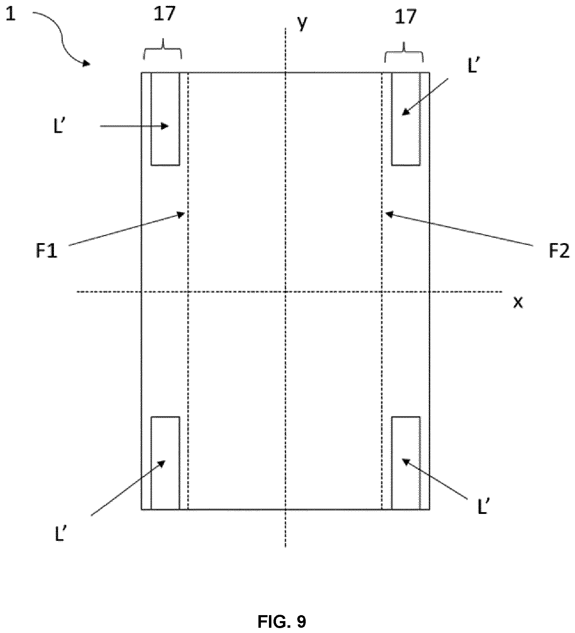

In a further embodiment, as shown in Fig. 9, the fastening means (10) comprises at least four longitudinally extending sections (L'), one longitudinal extending section in each of the four quadrants of the insert (1). In an alternative embodiment, the fastening means (10) comprises at least two laterally extending sections (T), one lateral extending section in each of the first and second transverse half proximal the first and second transverse edges (6,7).

In a further embodiment, the fastening means (10) have a higher resistance to bending and/or higher stiffness compared to the backsheet (3) of the insert (1). The latter difference gives the insert (1) a certain stability/rigidity when attaching it to the re-usable outer shell (20).

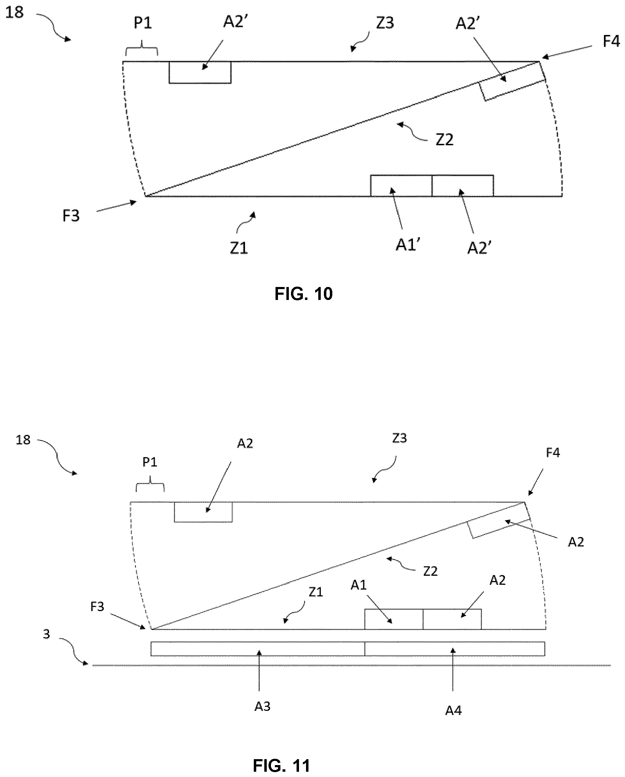

In an embodiment, shown in Fig. 10, the fastening means (10) comprise a Z-folded portion (18). Such configuration allows for a dual-use function in a way that the fastening means, by combining one or more adhesive areas (A1') and one or more mechanical areas (A2') onto different zones of the Z-folded fastening means, provides improved attachment in use to the re-usable outer shell (20) and provides means to dispose the insert in an easy and efficient manner. In such embodiment the tape takes a Z-form, being folded twice, once about F3 and a second time about F4. The Z-folded tape consists of zones (Z1, Z2 and Z3), each having one or more adhesive areas and/or one or more mechanical areas.

In a preferred embodiment, the first zone (Z1), zone closest to the garment-facing surface of the insert backsheet (3), consists of one or more adhesive areas and one or more mechanical areas, optionally alternatingly arranged or arranged in a mesh structure. The second zone (Z2) includes one or more mechanical areas and the third zones (Z3), being farthest away from the garment-facing surface of the insert backsheet (3), includes one or more mechanical areas. The Z-folded tape further comprises a section which can serve as a fingerlift (P1). Pulling the tape at the fingerlift section, allows to unfold the tape and attach zone 3, through the one or more mechanical areas, to the garment facing surface of the insert backsheet (3). This frees up the fastening means of zones 1 and 2 which serve to removably attach the disposable insert (1) to the re-usable outer shell (20). Upon disposal, the fingerlift (P1) can be used to detach zones 2 and 3 from the insert after which the one or more mechanical areas of zone 2 can be used to keep the rolled-up insert in such state for efficient disposal. In an alternative embodiment, Z2 and Z3 can comprise one or more adhesive areas; or one or more mechanical areas and one or more adhesive areas.

In an embodiment, shown in Fig.11, the attachment of the fastening means, in Z-folded state, to the garment-facing side of the disposable insert (1) is with adhesive. The adhesive can be divided into 2 zones (A3 and A4) wherein the peel strength between the fastening means, in Z-folded state, and the disposable insert (1) in zone A3 is less than the peel strength in zone A4. Such configuration allows the user to further extend the fastening means upon disposal if needed. By pulling the fingerlift (P1) bonding in zone A3 can break whereas the bonding in zone A4 stays intact due to the difference in peel strength.

In an embodiment the one or more mechanical areas comprise hooks. In an alternative embodiment the one or more mechanical areas comprise fiber fasteners as described herein.

Inserts (1) herein preferably comprise a pair of barrier cuffs extending along the first and second longitudinal edges (8, 9) and arranged to provide lateral barriers preventing exudate leakage. Typically, the barrier cuffs comprising a hydrophobic nonwoven and one or more elastics at an apex position thereof such to form standing gathers when the insert is extended for placement within the re-usable outer shell.

Generally, inserts herein are free of transversely extending side panels such as elastic side panels.

Backsheets for use herein are as commonly known in the art and may be breathable and/or comprise a laminate of a film, preferably polyethylene (PE) based or bio-PE based, and a nonwoven layer with the nonwoven layer being positioned on the outermost surface at a garment facing side thereof. Preferably, the backsheet (3), being a single layer or a laminate, is liquid impermeable. In an embodiment, the nonwoven layer joined to a garment-facing surface of the backsheet (3) preferably is corrugated or embossed such that a plurality of peaks and valleys are formed onto which the fastening means (30 and 80) may adhere. This arrangement advantageously allows for a stronger releasable connection of the insert (1) to the one or more patches (70) and/or to the re-usable outer shell (20).

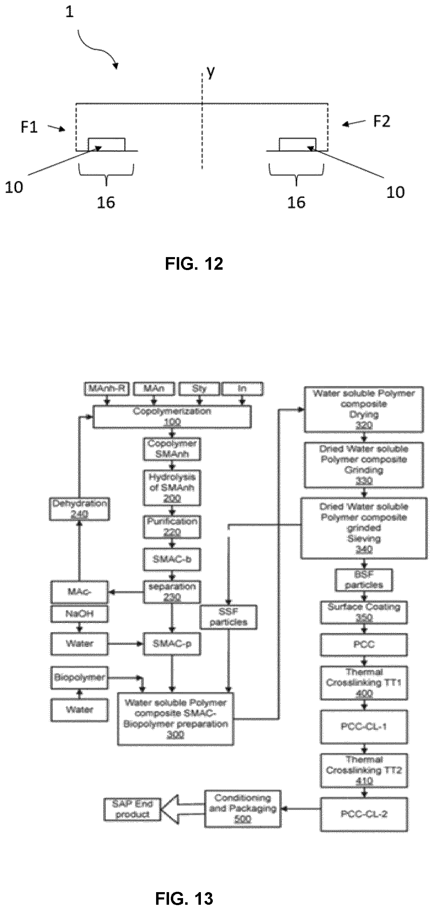

In an embodiment, as shown in Fig. 9 and 12, the insert (1) comprises folded sections (16) wherein longitudinally extending side margins (17) of the insert are folded to the garment-facing surface of the backsheet. Preferably, fastening means (10) are positioned on the garment-facing surface of the backsheet within these side margins. In this way, the fastening means are facing the garment-facing surface of the backsheet. This avoids multiple, disposable inserts adhering to each other which would inhibit efficient use of the inserts. Positioning the fastening means (10) in the side margins, proximal to the first and second transverse edge, provides for corner control during attachment of the disposable insert (1) to the re-usable outer shell (20).

The inserts (1) herein may be substantially rectangular in shape but may equally be shaped such as hourglass and/or substantially T-shaped. Other anatomical shapes are further contemplated herein and suitable in the present disclosure.

In an embodiment, the absorbent material comprises, preferably consists of, superabsorbent particles and/or cellulose fibers.

The absorbent cores herein may comprise at least 60%wt of superabsorbent particles, in particular at least 70%wt, preferably at least 80%wt, preferably at least 90%wt, by total weight of the core.

In an embodiment, the superabsorbent particles comprise a blend of superabsorbent particles comprising a first superabsorbent particles (SAP1) and a second superabsorbent particles (SAP2), wherein the first superabsorbent particles (SAP1) have an AUL that is greater than the AUL of the second superabsorbent particles (SAP2), and wherein the first superabsorbent particles (SAP1) have an AUL of greater than 15 g/g, according to the test method herein, preferably wherein the first superabsorbent particles (SAP1) have a particle size distribution that is greater than that of the second superabsorbent particles (SAP2). Preferably wherein at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles.

Preferably the second superabsorbent particles (SAP2) comprises, preferably consists of, Low-AUL Bio-SAP. "Low-AUL Bio-SAPs" means that they generally have AUL (as measured according to the test method herein) of less than 20 g/g, typically less than 15 g/g, and typically belong to the following classes: (a) materials consisting only of crosslinked biopolymers; (b) materials consisting only of crosslinked synthetic polymers; (c) composite materials consisting of synthetic polymers and biopolymers in certain variations: in inter crosslinking type connection with or without chemical agents; graft type; intercomplexate type and interpenetrate type. Copolymers such as styrene maleic acid are typically used in copolymerization processes for achieving Low-AUL Bio-SAP. Other exemplary sources include alpha-1,3 glucan, starch, starch and/or sodium salts, sugar and derivatives. Exemplary commercially available Low-AUL Bio-SAPs for use herein include: SAPs derived from charged-modified starches as sold from TETHIS 5237 Capital Blvd.; Raleigh, NC 27616, USA and further exemplified in US20200054782 A1 herein incorporated by reference; SAPs comprising a 100% acrylic acid co-acrylamide with little to no cross link shell, and the like all generally having AUL of less than 20 g/g according to the test method herein.

Preferably, the first superabsorbent particles (SAP1) are free of Low-AUL Bio-SAP. Advantageously this limits rewet drawbacks as described herein.

In an embodiment, the SAP1 is a High-AUL Bio-SAP "composite polymer" (i.e. a biodegradable superabsorbent composite polymer having an AUL of greater than 15 g/g, preferably greater than 20 g/g, as will be described in more detail herein below). The composite polymer may comprise a synthetic hydrophobic polymer and a natural biopolymer. More specifically, the composite polymer may comprise styrene maleic acid copolymer and a biopolymer of animal or vegetal origin in conformity with the technological flow chart presented in Fig. 5. Styrene maleic acid copolymer is preferably in salt form, more preferably in the form of monovalent cation salt. Alternatively, the High-AUL Bio-SAP, although less preferred, may comprise "non-composite polymers" and rather be selected from certified biomass superabsorbent polymers having AUL of greater than 15 g/g, preferably greater than 20 g/g, and typically certified by REDcert2 (https://www.redcert.org/images/SP_RC%C2%B2_Biomass-balanced_products_V1.0.pdf ). An example may be a polyacrylic acid, sodium salt, crosslinked, partly neutralized SAP from up to 100% allocated biomass feedstock such as commercially available HySorb® B 6600MB manufactured and sold by BASF SE, Ludwigshafen, Germany.

In an embodiment of the High-AUL Bio-SAP "composite polymer", the styrene moiety in the synthetic polymer can be replaced by other hydrophobic moieties. Exemplary synthetic polymers are: poly(maleic anhydride-co- methyl vinyl ether) (Gantrez), poly(vinyl chloride-co-maleic acid) and poly[(maleic anhydride)- alt-(vinyl acetate).

The term "composite" as herein used refers to a polymeric substance that a) is formed from at least two polymers with different macromolecular chemical structure; and b) the resulting composite is a unique entity that does not separate spontaneously to its components during application. It is understood that the term "composite" may include other substances such as drugs, stimulators, inhibitors, odorants, emollients, plasticizer and others.

The term "anionic" refers to a polymeric composite generating in aqueous media a negative electrochemical potential as the result of the presence in its structure of some free acid functional groups capable of dissociating into anions.

In an embodiment described in Fig. 13, the production process starts with the synthesis of copolymer (styrene-alt-maleic anhydride) by bulk co-polymerization using an excess of about 60-90% of maleic anhydride relative to styrene, whereas maleic anhydride works also as a solvent (operation 100).

Copolymerization is typically executed in Sigma-type mixer machines named Hermetic machines in order to be able to work at high pressures e.g. not exceeding 10 bar or in vacuum conditions (less than IOmbar) with double mantle as well with arms equipped with a heating - cooling system.

The copolymerization process for the production of a superabsorbent polymer typically uses styrene monomer stabilized with organic compounds that inhibit the process of homopolymerization during storage and transportation. Such inhibitors are for example substances such as : amino derivatives, thiols derivatives, and hydroxyl derivates as for example (2-hydroxypropyl)-ethylene diamine compounds, 4-tert-butylcatechol and others) being preferred 4-tert-butylcatechol in proportion of 0.002- 0.008% to the monomer, more preferably is 0.003-0.007% to the styrene and most preferably 0.004-0.006% to the styrene and the molar fraction of styrene in the reaction mass is 0.05-0.08, preferably 0.1-0.15 and more preferably 0.18-0.21.

The copolymerization may also contain maleic anhydride that is either fresh maleic anhydride MAnh or recovered maleic anhydride MAnh-R that is recycled from a previous batch as schematically showed in Fig. 5. and the amount of fresh maleic anhydride MAnh relative to recovered maleic anhydride MAnh-R is about 10 - 40% (dry basis).

For the copolymerization, further customary agents may be used such as peroxides, azo compounds etc., which form free radicals by thermal decomposition, while the quantity of initiator is 0.05- 0.15 %, preferably 0.07-0.009% and most preferably 0.08-0.12% to a double quantity of styrene adopted for copolymerization.

Next, it may follow the conversion of styrene-alt maleic anhydride copolymer to styrene-alt maleic acid copolymer by hydrolysis with water (process 200) which is done in the same equipment where copolymerization has been made. Conversion of styrene-alt maleic anhydride copolymer to styrene-alt maleic acid copolymer by hydrolysis using a quantity of water higher than the one stoichiometrical required for total hydrolysis (Ws) with an excess which represents 2-3% versus to the stoichiometrical value, preferably in excess of 4-5% to the stoichiometrical water and most preferably 5-10% in excess to the stoichiometrical water.

The total quantity of water necessary to styrene and maleic anhydride copolymer's hydrolysis is typically inserted in the mass of reaction in two steps from which 50% is in the form of 0.005N hydrochloric acid solution and the rest as non-acidulated water.

The acidulated water is typically inserted into the reaction mass in two portions at a mass reaction's temperature that not exceeding 60°C at 15 minutes intervals between each dosage, and the quantity of non-acidulated water is inserted into the mass of reaction also in two portions, from which the first is after 60 minutes from the last portion of acidulated water, then is inserted the last portion of non-acidulated water and mixing of reaction mass for 45-60 minutes in cooling conditions of 35-40°C. Reaction mass resulted after hydrolysis is a wet solid in form of a powdery mass of white colour with a value of bulk density of 0.6-0.8 g/cm3. Further, mass of reaction that resulted after copolymerization and hydrolysis (which is a blend of styrene maleic acid copolymer - SMAC and free maleic acid -MAC, which contains traces amounts of non-reacted styrene and stabilizer for styrene as well as traces of hydrochloric acid) is transferred to perform the purification process of styrene maleic acid copolymer (process 220).

The purification process which represents the extraction of free maleic acid fraction from SMAC polymer mass is typically done in an equipment such as tank-type with mixing in which over reaction mass resulted after hydrolysis of synthetic copolymer is added a quantity of deionized water for purification [Wp] that represents a quantity correlated to the wet mass of reactions (RM) in accordance with the relationship Wp= 2 * RM or Wp= 5 * RM, preferably Wp= 3 * RM.

Purification generally consists of 3-5 extraction stages followed each time by filtration. The number of extraction and filtration operations are established so that the content of the free carboxylic groups found in polymer of SMAC to be between 0.00909-0.0095 mole/gram, preferably between 0.0091-0.0094 mole/gram and most preferably between 0.0092- 0.0093 mole/gram.

The extraction in fact preferably occurs at temperature of 60°C for 30 minutes and each filtration (process 230) is done with known equipment as press filter or Nuce filter. All solutions resulted from filtering are collected into a tank of supernatant in order to process the maleic acid which it contain.

The processing of maleic acid water solution to recovery of maleic anhydride preferably consists of the following operations: a) concentration of maleic acid solution through reverse osmosis; b) spray drying of maleic acid concentrated solution when resulted maleic acid powder and water; c) conversion of maleic acid powder to maleic anhydride by thermal-vacuum dehydration (with technological parameters modified than those mentioned in US Pat No.4,414,398 when in the end is obtained a material called recovered maleic anhydride (MAnh-R). The purified filtrate of SMAC polymer is typically collected in an equipment as Sigma mixer type in order to be processed with biopolymers to obtain the water soluble composite polymer containing synthetic SMAC polymer and biopolymer [WSPC] (process 300).

The preparation process of polymeric composite [WSPC] containing SMAC and a biopolymer of animal or vegetal origin is preferably preceded by other operations as follows: a) Preparation of a base solution is obtained by dissolution of a solid hydroxide compound in water to 40% by weight, whereas examples of preferred base hydroxide compounds are sodium hydroxide, lithium hydroxide, potassium hydroxide, ammonium hydroxide, preferably sodium hydroxide; b) transformation of styrene maleic acid copolymer obtained in step 230 into styrene-maleic acid monovalent cation salt by neutralization with base solution prepared in a) above in order to obtain a solution of copolymer salt with concentration higher than 25% preferably higher than 35 % and most preferably higher than 50%. Neutralization of the SMAC copolymer is 48-58%, preferably 50-56% and most preferably 52-54%; c) preparation of the biopolymer (as gelatin, albumin, casein, soy, guar or starch, preferably is gelatin) as water solution of 40% by weight in water; d) preparation of polymeric composite is done by treating the solution of styrene maleic acid salt with biopolymer solution at temperature of 55-75°C for 30 minutes.

The amount of biopolymer relative to copolymer in the composite is preferably from 4-6 % (dry basis), preferably 8-10 % (dry basis) and most preferably 12-14 % (dry basis).

The blending of the composite mass typically continues during 4-5 hours until the polymeric mass is transformed from the viscous solution into a partially dried granular mass with a moisture content not higher than 20%. This partially dried polymeric composite material WSPC in granular form is typically subjected to a supplementary drying (process 320) at temperatures of preferably 75-85°C on conveyor belt type or Rotary type in order to obtain final drying of until the moisture content is less than 14%, preferably less than 12% and most preferably less than 8%.

Further steps of drying, grinding and sieving are preferably carried out (process 330 + process 340) to obtain two types of solid phases called herein large solid phase (LSP) with granulometric distribution higher than 100 microns, preferably of 100 -850 microns that corresponds to be used in the manufacture of diapers and a small solid phase (SSF) with granulometric distribution up to 100 microns. The small solid phase SSF is re-used in the preparation of the next new batch of SAP that comes into the process 300.

The large solid phase LSP may be exposed to post-treatment surface coating processes using known chemical substances as: glycerin ethylene glycol, propylene glycol or polyether hydroxyl with properties of biodegradability. Examples of preferred coating materials are hydroxyl polyether, more preferably polyethylene glycol - PEG 200 used at a rate of 0.2-2% by weight (dry basis) to LSP, preferably at a rate of 0.5-1.5% by weight and most preferably at a rate of 0.8-1.2% by weight to LSP.

Surface coating may be applied using equipment like powder coating machines at temperatures of generally 30-70°C, preferably at temperatures of 35-65°C and most preferably at temperatures of 40-60°C for 30-90 minutes, preferably for 40-75 minutes and most preferably for 50-60 minutes. As a result of this process is resulted the material called Polymer Composite Coated Treated- PCC (operation 350).

The obtained material after surface coating (Polymer Composite Coated) may be subdue to a thermal treatment called first thermal treatment (TT1) (operation 400) consisting in warming of particles mass in hot air with temperature of 90-I40°C for 30-150 minutes, preferably with temperatures of 100-135°C for 45-120 minutes and most preferably by bulk thermal crosslinking at temperatures of 110-120°C, for 60-90 minute using equipment of conveyor belt type or shaking rotary type when is resulted an intermediary material called Polymer Composite Coated first crosslinked (PCC-CL-I).

The material PCC-CL-I may be subdue to a new thermal treatment (TT2) (operation 410) in hot air with temperature of 120-150°C for 5-30 minutes, preferably at temperatures of 125-145°C for 10-25 minutes and most preferably to temperature of 120-140°C for 15-20 minute in the same type of equipment used for TT1 and is obtained the Polymer Composite Coated second crosslinked (PCC-CL-2). The material (PCC-CL-2) may then be conditioned for 24 hours in an atmosphere in which the air has a moisture content of 65% and temperature of 20°C and then is packaged in sealed polyethylene bags (operation 500).

The material resulting after conditioning is a biodegradable SAP with AUL higher than 20g/g in aqueous solution of 0.9% NaCl at pressure of 0.9 psi, which represents the end product of the manufacturing process which is the object of the present invention, i.e. the "High-AUL biodegradable superabsorbent composite polymer" referred to herein.

Reference is made to co-pending application EP21152698.3 for further examples of Low-AUL Bio-SAP and High-AUL Bio-SAPs that may be used in absorbent cores herein, with particular reference to Example 1.

In an embodiment, the first superabsorbent particles (SAP1) are comprised at a level of less than 80%wt, preferably from 10%wt to 32%wt; and the second superabsorbent particles (SAP2) are comprised at a level of at least 20%wt, preferably from 25%wt to 100%wt, more preferably from 30% to 90%, even more preferably from 35% to 80%, even more preferably from 40% to 70%, even more preferably from 45% to 68%, by total weight of superabsorbent particles.

Preferably, the AUL ratio AULSAP1/AULSAP2 of the first superabsorbent particles (SAP1) and second superabsorbent particles (SAP2) is greater than 1.4, preferably greater than 1.5, more preferably from 1.6 to 5, even more preferably from 1.7 to 3. Advantageously, this allows to ensure the right balance of reduced rewet and fast liquid absorption especially on the lower layer and optimal performance under load.

In an embodiment, the first superabsorbent particles (SAP1) have a lower absorption speed (typically according to the vortex method herein) than the second superabsorbent particles (SAP2). Preferably, the vortex time (according to the vortex method herein) of SAP1 is more than 50 seconds, preferably from 60 seconds to 90 seconds. Preferably, the absorption speed of SAP2 is less than 45 seconds, preferably less than 40 seconds, even more preferably from 15 seconds to 35 seconds.

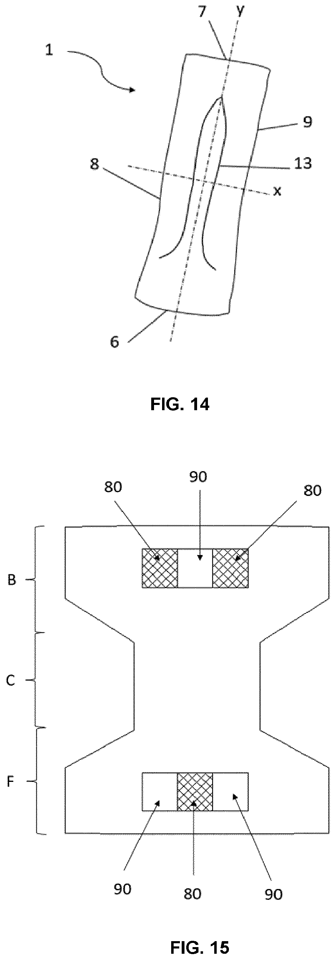

In an embodiment, as shown in Fig. 14, the absorbent core (4) comprises one or more attachment zones wherein a top layer of a core wrap (14) is adhered to a lower layer of a core wrap (15) such that one or more channels (13) substantially free of absorbent material are formed, preferably wherein the wetness indicator (10) extends between said channels (13) and preferably overlaps at least a portion of said channels. The channels (13) may extend from 10% to 85%, preferably from 15% to 75%, of a length of the core (4) extending in a direction substantially parallel to the longitudinal axis (y).

In a preferred embodiment, channels (13) herein are longitudinally extending and cross both the transverse centerline (x) at a first crossing point and the longitudinal centerline (y) at a second crossing point, wherein the first and second crossing points are different and preferably wherein the second crossing point is positioned closer to the second transverse edge (7) than the first crossing point, and preferably wherein the second transverse edge (7) is positioned proximal to the back region (B) of the outer shell (20) when said insert (1) is connected thereto. Advantageously this allows for improved cup formation in a hybrid concept as well as retaining liquid distribution advantages by guiding exudates to the back of the core.

In a preferred embodiment, one or more of the patches (70) are releasably attached to the front waist region (F) of the re-usable outer shell (20) and one or more of the patches (70) are releasably attached to the back waist region (B) of the re-usable outer shell (20). The one or more patches (70) releasably attachable to the front waist region (F) comprise alternating zones of fastening means (80) and loop components (90) and the one or more patches releasably attachable to the back waist region comprise substantially complementary alternating zones of fastening means (80) and loop components (90). An example is shown in Fig.15 wherein the fastening means (80) in the front waist region (F) are releasably attachable to the loop components (90) in the back waist region (B) and wherein the fastening means in the back waist region (B) are releasably attachable to the loop components (90) in the front waist region (F). Advantageously this allows for the user to couple the front waist region (F) to the back waist region (B) before washing or allows the user to store away the re-usable outer shell in a compact manner.

There may be provided a kit of parts comprising: a plurality of patches (70) and at least one of: (i) one or more outer shells (20) forming a plurality of absorbent assemblies. Preferably, the plurality of inserts (1) differ in at least one of: size, absorbency, biodegradable and/or compostable content, shape, and number of components contained therein. Advantageously this allows for different inserts targeting different types of usage e.g. day vs night, size increases of subjects/wearers etc. whilst retaining substantially the same outer shell. (ii) one or more inserts (1) forming a part of a plurality of absorbent assemblies and one or more outer shells (20). Preferably, the plurality of outer shells (20) differ in at least one of: size, absorbency, biodegradable and/or compostable content, shape, and number of components contained therein.Advantageously this allows for different outer shells targeting different types of usage e.g. day vs night, size increases of subjects/wearers etc. whilst retaining substantially the same insert type.

Such kits may allow for extended use of the re-usable outer shells.

TEST METHODS

AUL (Absorbency Under Load, 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as W0. Then 0.900 +/- 0.005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

$$AUL0.7psig/g=Wb-Wa/Wa-W0$$

AUL 0.3 psi and 0.5 psi are measured similarly at the appropriate lower pressure.

Absorption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:

1. Beaker, 100 ml.
2. Programmable magnetic stir plate, capable of providing 600 revolutions per minute.
3. Magnetic stir bar without rings, 7.9 mm x 32 mm, Teflon covered.
4. Stopwatch.
5. Balance, accurate to $\pm$ 0.01 g.
6. Saline solution, 0.9%.
7. Weighing paper.
8. Room with standard condition atmosphere: Temp = 23°C $\pm$ 1°C and Relative Humidity = 50% $\pm$ 2%.

Test procedure:

1. Measure 50 g $\pm$ 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g $\pm$ 0.01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

It is supposed that the present invention is not restricted to any form of realization described previously and that some modifications can be added to the presented embodiments without reappraisal of the appended claims.

**Claims**

1. A patch (70) comprising:

a base layer including a body-facing surface and a garment-facing surface; the base layer comprising a perimeter formed by a first and second transverse edge (71 and 72) and first and second longitudinal edges (73 and 74) connecting the first and second transverse edges; the garment-facing surface comprising a fastening means (60) for releasable attachment to a re-usable outer shell (20), the body-facing surface comprising a fastening means (80) for releasable attachment to a disposable insert (1).

2. A patch (70) according to Claim 1 wherein the fastening means (60) comprises one or more snap fasteners, selected from male snap fasteners (62) and/or female snap fasteners (61).

3. A patch (70) according to any of the preceding Claims wherein the fastening means (80) comprises one or more mechanical areas (A2) and/or one or more adhesive areas (A1).

4. A patch (70) according to any of the preceding Claims comprising alternating zones of fastening means (80) and loop components (90), wherein the length ratio (L1/L) of the fastening means (80) is more than about 0,1; more preferably from about 0,1 to about 0,9 and wherein the length ratio (L2/L) of the loop components (90) is more than about 0,1; more preferably from about 0,1 to about 0,9.

5. A patch (70) according to Claim 3-4 wherein the total area ratio ($\Sigma$A1 / $\Sigma$A2) of the fastening means (80) is from about 0,4 to about 1, preferably from about 0,45 to about 0,8; more preferably about 0,5.

6. A re-usable outer shell (20) comprising:

a front (F) and back (B) waist region having uppermost and lowermost edges; and a crotch region (C) interposed between the front (F) and back (B) waist region, wherein the uppermost edges of the waist region form a waist opening and the lowermost edges of the waist region together with lateral extremities of the crotch region form two oppositely disposed leg openings; and wherein at least the body-facing surface of the front (F) and/or back (B) waist region(s) is adapted for receiving one or more discrete patches (70), such as that of any of Claims 1 to 5; and wherein the re-usable outer shell (20) comprises fastening means (90) on the body-facing surface including one or more snap fasteners (60), selected from male snap fasteners (62) and/or female snap fasteners (61), for releasably engaging female and/or male snap fastener(s) of the discrete patches (70).

7. A re-usable outer shell (20) according to claim 6 comprising re-fastenable or permanent side seams (21, 21') joining the front (F) and back waist (B) regions, said outer shell (20) preferably being in the form of a pant; or wherein the back waist region (B) comprises, preferably two, transversely extending side panels (22, 22') each comprising a fastener (23, 23') for coupling to a landing zone positioned at the garment facing side of the front waist region (F), said outer shell (20) preferably being in the form of a diaper.

8. A re-usable outer shell (20) according to any of claims 6-7 wherein the front waist region comprises one or more male snap fasteners (62) and the back waist region comprises one or more female snap fasteners (61); or wherein the front waist region comprises one or more female snap fasteners (61) and the back waist region comprises one or more male snap fasteners (62).

9. An absorbent assembly comprising:

> one or more patches (70) according to any of Claims 1 to 5;
> an outer shell (20) according to any of Claims 6 to 8; and a disposable insert (1)

10. An absorbent assembly according to Claim 9 wherein one or more of the patches (70) are removably joinable to the front waist region (F) of the re-usable outer shell (20) and one or more of the patches (70) are removably joinable to the back waist region (B) of the re-usable outer shell (20); wherein the one or more patches (70) releasably attached to the front waist region (F) comprise alternating zones of fastening means (80) and loop components (90) and the one or more patches (70) releasably attachable to the back waist region comprise substantially complementary alternating zones of fastening means (80) and loop components (90).

11. An absorbent assembly according to Claim 10 wherein the disposable absorbent insert (1) comprises:

> a liquid permeable topsheet (2) ;
> a backsheet (3) ; and
> an absorbent core (4) comprising absorbent material (5) therein and being sandwiched between said topsheet (2) and backsheet (3);
> wherein the insert (1) comprises a perimeter formed by first and second transverse edges (6, 7) and first and second longitudinal edges (8, 9) connecting the first and second transverse edges (6, 7); a longitudinal centerline (y) extending substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7), thereby forming four quadrants; wherein the disposable absorbent insert (1) is releasably attached to the one or more patches (70).

12. An absorbent assembly according to Claims 10-11 wherein the insert (1) comprises a backsheet (3), being a laminate of a nonwoven layer and a film layer; the nonwoven layer being positioned opposite the body-facing surface of the one or more patches (70).

13. An absorbent assembly according to Claim 12 wherein the nonwoven layer of the backsheet (3) comprises an embossing pattern.

14. An absorbent assembly according to Claims 10-13 wherein the insert (1) comprises fastening means (10) including one or more adhesive areas (A1) and one or more mechanical areas (A2); wherein the total area ratio ($\Sigma A1$ / $\Sigma A2$) of the fastening means (10) is from about 0,3 to about 1, preferably from about 0,45 to about 0,8; more preferably about 0,5; the insert (1) being releasably attachable to a re-usable outer shell (20).

15. An absorbent assembly according to Claim 14 wherein the fastening means (10) comprises a Z-folded portion (18).

16. A kit of parts comprising:

> a plurality of patches (70) according to any of Claims 1 to 5; and
> at least one of:

>> (i) one or more outer shells (20) according to any of Claims 6 to 8, forming a plurality of absorbent assemblies according to Claims 11 to 15; preferably wherein the plurality of inserts (1) differ in at least one of: size, absorbency, biodegradable and/or compostable content, shape, and number of components contained therein; and
>> (ii) one or more inserts forming a part of a plurality of absorbent assemblies according to Claims 11 to 15; one or more outer shells (20) according to any of Claims 6 to 8; preferably wherein the plurality of outer shells (20) differ in at least one of: size, absorbency, biodegradable and/or compostable content, shape, and number of components contained therein.

**FIG. 1**

**FIG. 2**

FIG. 3

FIG. 4

FIG. 5

FIG. 6

**FIG. 7**

**FIG. 8**

**FIG. 9**

**FIG. 10**

**FIG. 11**

**FIG. 12**

**FIG. 13**

**FIG. 14**

**FIG. 15**

Europäisches
Patentamt

European
Patent Office

Office européen
des brevets

# EUROPEAN SEARCH REPORT

**Application Number**

EP 22 17 3885

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CA 2 103 537 A1 (LEE PETER H T [CA]) 7 February 1995 (1995-02-07) * paragraphs [0010] – [0012] * * figures 1,8 * | 1-6,9-16 | INV. A61F13/15 A61F13/49 A61F13/505 A61F13/58 |
| X | US 2016/278999 A1 (WANG BRANDON SHUI LING [HK] ET AL) 29 September 2016 (2016-09-29) * paragraphs [0153], [0154], [0158], [0160], [0165]; figures 26-32B * | 1-3,6-9, 11,12 | A61F13/62 A61F13/74 |
| X | EP 3 906 905 A1 (ONTEX BV [BE]; ONTEX GROUP NV [BE]) 10 November 2021 (2021-11-10) | 1,3 | |
| A | * paragraphs [0008], [0009], [0035] – [0037] * * paragraphs [0057] – [0060] * | 2,4-16 | |
| X | US 2014/257229 A1 (WANG SAMANTHA CHEN-YEE [US] ET AL) 11 September 2014 (2014-09-11) | 1 | |
| A | * paragraphs [0010], [0071], [0090], [0091] * * paragraphs [0100], [0101], [0102], [0211], [0213], [0219] * | 2-16 | TECHNICAL FIELDS SEARCHED (IPC) A61F |

The present search report has been drawn up for all claims

| Place of search | Date of completion of the search | Examiner |
|---|---|---|
| Munich | 9 November 2022 | Beins, Ulrika |

EPO FORM 1503 03.82 (P04C01)

## ANNEX TO THE EUROPEAN SEARCH REPORT
## ON EUROPEAN PATENT APPLICATION NO.

**EP 22 17 3885**

This annex lists the patent family members relating to the patent documents cited in the above-mentioned European search report.
The members are as contained in the European Patent Office EDP file on
The European Patent Office is in no way liable for these particulars which are merely given for the purpose of information.

**09-11-2022**

| Patent document cited in search report | | Publication date | Patent family member(s) | | Publication date |
|---|---|---|---|---|---|
| CA 2103537 | A1 | 07-02-1995 | NONE | | |
| US 2016278999 | A1 | 29-09-2016 | AU | 2016224987 A1 | 12-10-2017 |
| | | | BR | 112017018411 A2 | 17-07-2018 |
| | | | CA | 2977982 A1 | 01-09-2016 |
| | | | CN | 107847368 A | 27-03-2018 |
| | | | EP | 3261598 A1 | 03-01-2018 |
| | | | HK | 1252382 A1 | 24-05-2019 |
| | | | JP | 7004572 B2 | 10-02-2022 |
| | | | JP | 2018506390 A | 08-03-2018 |
| | | | JP | 2022058479 A | 12-04-2022 |
| | | | KR | 20170132756 A | 04-12-2017 |
| | | | MX | 370845 B | 08-01-2020 |
| | | | MY | 181698 A | 04-01-2021 |
| | | | PH | 12017501533 A1 | 05-02-2018 |
| | | | PL | 3261598 T3 | 18-05-2020 |
| | | | RU | 2017133108 A | 26-03-2019 |
| | | | SG | 10202109759P A | 28-10-2021 |
| | | | SG | 11201706894Y A | 30-10-2017 |
| | | | US | 2016278999 A1 | 29-09-2016 |
| | | | US | 2020113750 A1 | 16-04-2020 |
| | | | WO | 2016138466 A1 | 01-09-2016 |
| EP 3906905 | A1 | 10-11-2021 | NONE | | |
| US 2014257229 | A1 | 11-09-2014 | CN | 105007876 A | 28-10-2015 |
| | | | EP | 2964176 A1 | 13-01-2016 |
| | | | JP | 2016512060 A | 25-04-2016 |
| | | | US | 2014257229 A1 | 11-09-2014 |
| | | | WO | 2014138274 A1 | 12-09-2014 |

EPO FORM P0459

For more details about this annex : see Official Journal of the European Patent Office, No. 12/82

**EP 4 268 778 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3842017 A1
- EP 3659563 A1
- EP 3895673 A1
- US 20200054782 A1
- US 4414398 A
- EP 21152698